Europäisches Patentamt

**European Patent Office**

**Office européen des brevets**

(19)

(11) Veröffentlichungsnummer: **0 017 728**
**A2**

(12) # EUROPÄISCHE PATENTANMELDUNG

(21) Anmeldenummer: **80100830.1**

(51) Int. Cl.³: **A 61 B 5/14**

(22) Anmeldetag: **20.02.80**

(30) Priorität: **07.03.79 DE 2908817**
**19.11.79 DE 2946660**

(43) Veröffentlichungstag der Anmeldung: **29.10.80**
**Patentblatt 80/22**

(84) Benannte Vertragsstaaten: **AT BE CH FR GB IT LU NL SE**

(71) Anmelder: **C.A. Greiner & Söhne GmbH & Co. KG,**
**Galgenbergstrasse 9c, D-7440 Nürtingen (DE)**

(72) Erfinder: **Feaster, William Wardock, L'Estoril Avenue**
**Princesse Grace, Monte Carlo (MC)**

(74) Vertreter: **Dreiss, Uwe, Dr. jur. Dipl.-Ing. M.Sc et al,**
**Patentanwälte Dreiss & Fuhlendorf Schickstrasse 2,**
**D-7000 Stuttgart 1 (DE)**

(54) **Blutentnahmegerät mit Blutprobenröhrchen und Verfahren zur Evakuierung des Röhrchens.**

(57) Blutentnahmegerät mit einem hohlzylindrischen an einem Ende offenen Halter (1) und einer doppelendigen hohlen Nadel (7) am anderen Ende, in das ein evakuiertes Blutprobenröhrchen (2) eingeschoben wird, bei dem das Blutprobenröhrchen durch eine mit einer Öffnung versehene aufschraubbare Kappe (17) verschlossen wird, wobei die Kappe eine die Dichtung bildende Membrane (18) abdichtend an das Blutprobenröhrchen andrückt. Ferner ein Gerät (19) zur Evakuierung derartiger Röhrchen. In dem verschlossenen Ende des Halters (101) kann eine zweite Nadel (134) vorgesehen sein, über die das eingeschobene Blutprobenröhrchen (2) mit einer Saugleitung (135) verbunden werden kann, die in einem Ausführungsbeispiel durch einen zusammendrückbaren elastischen Ballon (504, 515) gebildet wird.

PATENTANWÄLTE

# DREISS & FUHLENDORF

SCHICKSTR. 2, D-7000 STUTTGART 1

- 1 -

BEZEICHNUNG GEÄNDERT
siehe Titelseite

<u>Blutentnahmegerät</u>

Die Erfindung betrifft ein Blutentnahmegerät der im Oberbegriff des Patentanspruchs 1 genannten Art, sowie ferner ein Verfahren zu seiner Anwendung, und ein Gerät zur Durchführung eines zur Anwendung erforderlichen Verfahrensschrittes.

Blutentnahmegeräte der im Oberbegriff des Patentanspruchs 1 genannten Art sind bekannt (US-PSen 24 60 641, 31 36 440). Die Dichtung des Blutprobenröhrchens wird dabei durch einen Gummistopfen gebildet. Öffnet man diesen Stopfen, um die Blutentnahme der weiteren Verwendung bzw. Analyse zuzuführen, so entsteht an der Oberfläche der Blutprobe momentan ein gewisser Unterdruck, der dazu führt, daß beim Herausreißen des Gummistopfens einige Tropfen der Blutprobe verschüttet werden können. Dieser "Aerosol"-Effekt wird beim schnellen Aufreißen des Stopfens durch die Elastizität des Materials, aus dem der Stopfen ist, begünstigt. Besonders gravierend ist die Gefahr eines "Aerosol"-Effekts, wenn der Stopfen infolge einer anschließenden Handhabung des Blutprobenröhrchens an seiner inneren Oberfläche mit Probenflüssigkeit bedeckt ist; dann werden beim Aufreißen des Stopfens einzelne Tröpfchen von der Oberfläche regelrecht weggeschleudert. Dieser Effekt bringt Infektionsgefahren. Die Bedienungsperson kommt mit Patientenblut, das infiziert sein kann, in Berührung. Auch eine gegenseitige Verunreinigung (cross-contamination) von Proben, die dadurch im Labor verursacht sein kann, spielt eine große Rolle. Man hat aus diesem Grunde schon eigens Stopfenentferner als besondere Geräte entwickelt. Die Verwendung eines besonderen Werkzeugs dieser Art zum Öffnen der Röhrchen ist jedoch unpraktisch, selbst wenn das Klinikpersonal sich daran gewöhnt.

Da bei den bekannten Einrichtungen das Vakuum bereits bei der Produktion hergestellt wird, muß die Dichtung so ausgelegt sein, daß sie dies längere Zeit aufrecht erhält. Der Gummistopfen muß am Rand dick sein. Der Durchstich der Kanüle erfordert oft zu starken Kraftaufwand. Man sieht daher in der Mitte des Stopfens eine Mulde vor, um eine dünnere Stelle für den Durchstich zu haben. Daher kann man die Nadel nicht in Nähe des Randes, also exzentrisch anordnen, was an sich erwünscht wäre, um flach einstechen zu können. Man kann bei den bekannten Röhrchen ferner den Nachteil haben, daß, je nachdem, wie alt das Röhrchen ist, das Vakuum, auch bei anfänglich gleichem Wert, schon etwas abgebaut ist, so daß weder stets gleichbleibende Geschwindigkeit der Blutentnahme, noch stets gleiches Volumen der entnommenen Probe gewährleistet ist.

Der Erfindung liegt die Aufgabe zugrunde, ein Blutentnahmegerät der eingangs genannten Art zu schaffen, bei der die Gefahr eines solchen "Aerosol"-Effekts beim Öffnen des Blutproberöhrchens ausgeschaltet ist. Außerdem soll die Dichtung besonders einfach und zuverlässig und das Vakuum bei der Anwendung stets gleich sein.

Erfindungsgemäß wird dies durch die im Kennzeichen des Patentanspruchs 1 angegebenen Merkmale gelöst.

Die Erfindung löst also das genannte Problem auf besonders einfache Weise dadurch, daß ein relativ dünnes und einfaches Dichtungsplättchen, die Membran, mit Hilfe eines Schraubverschlusses als Dichtung verwendet wird. Damit wird erstmals ein Schraubverschluß bei den Vakuum-Blutentnahmesystemen der eingangs genannten Art einsetzbar, obwohl man seither den Gummistopfen wegen des Durchstechens mit der Nadel für unvermeidlich hielt. Dieses Vorurteil ist nun durch die Erfindung überwunden. Die Membran kann relativ dünn und/oder aus weicherem Material hergestellt sein, so daß der Durchstich leichter wird und nur noch etwa 1/3 der Kraft benötigt. Die Nadel kann dann auch exzentrisch angeordnet werden. Der zunächst befürchtete Nachteil, die Dichtung sei nicht ausreichend, wird dadurch überwunden, daß eine weitere extreme Vereinfachung der Anwendung dadurch erzielt wird, daß die Röhrchen zunächst überhaupt nicht evakuiert sein müssen, sondern eine Eva-

- 4 -

kuierung erst kurz vor der Anwendung erfolgt,
also z.B. am Anfang eines Tages für einen Tagesbedarf. Dies geschieht durch eine einfache Pumpe,
die ebenfalls über eine Nadel, mit der die Membran
durchstochen werden kann, mit dem Inneren des
Röhrchens verbunden werden kann. Das hat u.a. den
Vorteil, daß ein Blutprobenröhrchen, dessen Vakuum
aus Versehen abgebaut wurde (etwa durch einen unbeabsichtigten Durchstich), erneut evakuiert
werden kann.

Dabei ist ein einfacher konstruktiver Aufbau gewährleistet. Insbesondere ist der Aufbau einfach
im Vergleich zu Blutentnahmesystemen, die zur
Erzeugung eines Vakuums im Prinzip wie Spritzen
arbeiten.

Eine Weiterbildung betrifft dabei eine Blutentnahme unter Aspiration, d.h. bei Kontrolle des
Unterdrucks im Blutprobenröhrchen, in besonders
einfacher Weise. Dabei ist in einem Ausführungsbeispiel ein Gummiballon vorgesehen. Dann ist es
besonders einfach, durch Zusammendrücken und kontrolliertes Loslassen des Gummiballons den Unterdruck im Blutprobenröhrchen und damit die Geschwindigkeit, mit der einem Patienten Blut abgenommen wird, zu steuern. Dabei kann der Gummiballon gleichzeitig auch als Halter Verwendung
finden. Zwischen Gummiballon und der zweiten
Nadel, die zur Aspiration dient, ist ein Ventil
vorgesehen, das beim Zusammenpressen des Gummiballons verhindert, daß Druckluft zum Blutprobenröhrchen gelangt, andererseits beim Loslassen des
Gummiballons den Saug- oder Unterdruck, der dadurch entsteht, im Blutprobenröhrchen wirksam
werden läßt. Der Gummiballon kann auch fischblasenartig entlang der Außenseite des Halters und
an diesem anliegend ausgebildet sein. Auf diese
Weise ist die einhändige Handhabung besonders einfach.

Die Erfindung betrifft mehrere vorteilhafte
Weiterbildungen.

Ausführungsbeispiele der Erfindung werden im
folgenden anhand der beigefügten Zeichnungen beschrieben. Es stellen dar:

Figur    1    ein erstes Ausführungsbeispiel im
              Zustand vor der Anwendung;

Figur    1a   eine Seitenansicht des Ausführungs-
              beispiels nach Fig. 1;

Figur    2    das Ausführungsbeispiel nach Fig. 1
              bei der Anwendung;

Figur    3    eine Vorrichtung zur Evakuierung eines
              Blutprobenröhrchens;

Fig. 4a, 4b, 4c
              Draufsicht, Querschnitt und Seiten-

- 6 -

ansicht eines zweiten Ausführungsbeispiels;

Figur   5   ein drittes Ausführungsbeispiel;

Figur   6   ein viertes Ausführungsbeispiel;

Figur   7   ein fünftes Ausführungsbeispiel;

Figur   8   die Handhabung des Ausführungsbeispiels nach Fig. 7;

Figur   9   ein sechstes Ausführungsbeispiel
der Erfindung;

Figur   10   die Handhabung des Ausführungsbeispiels nach Fig. 9.

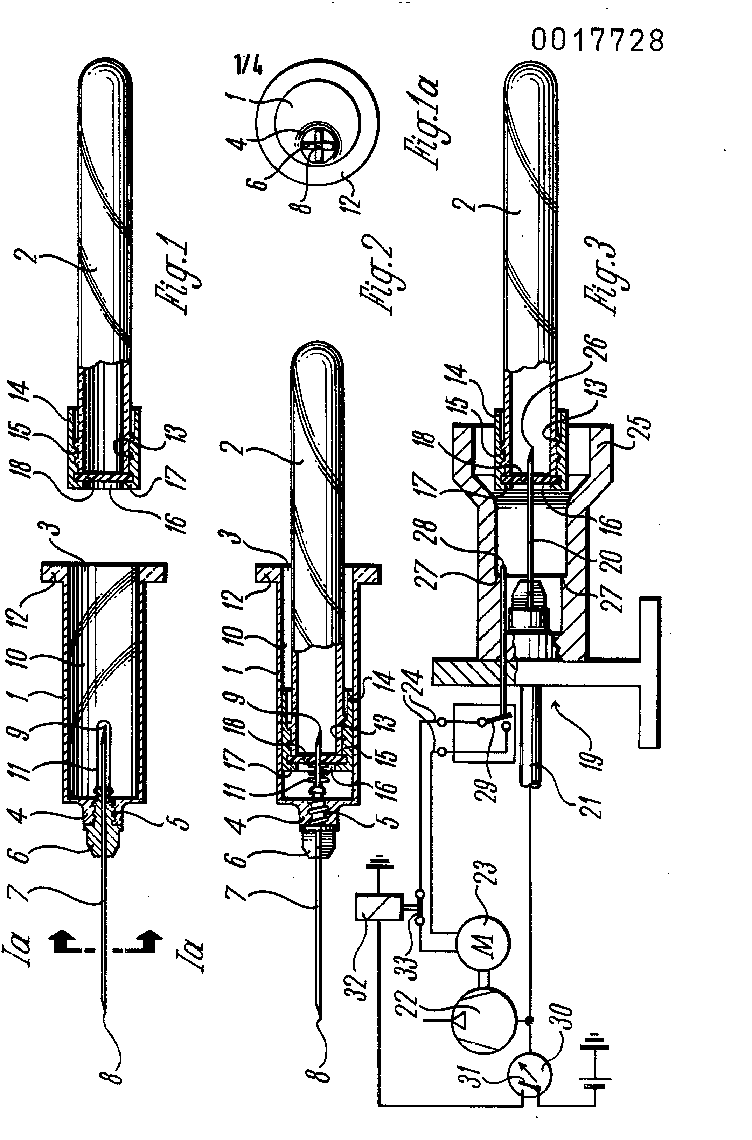

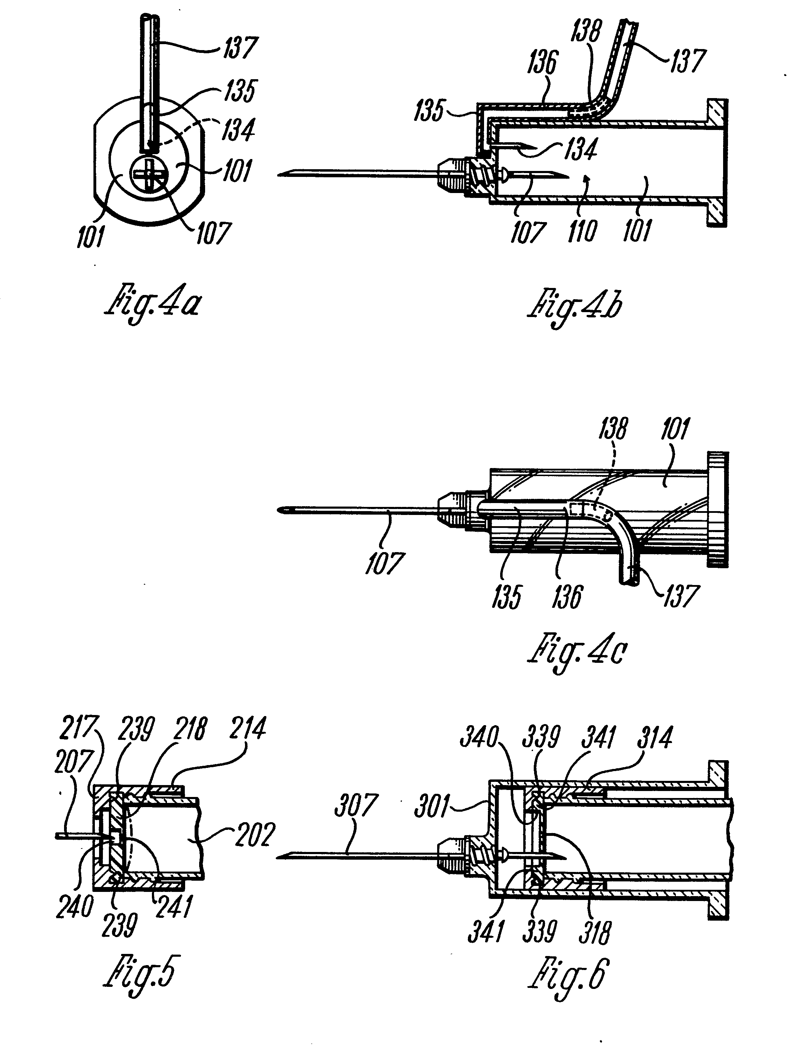

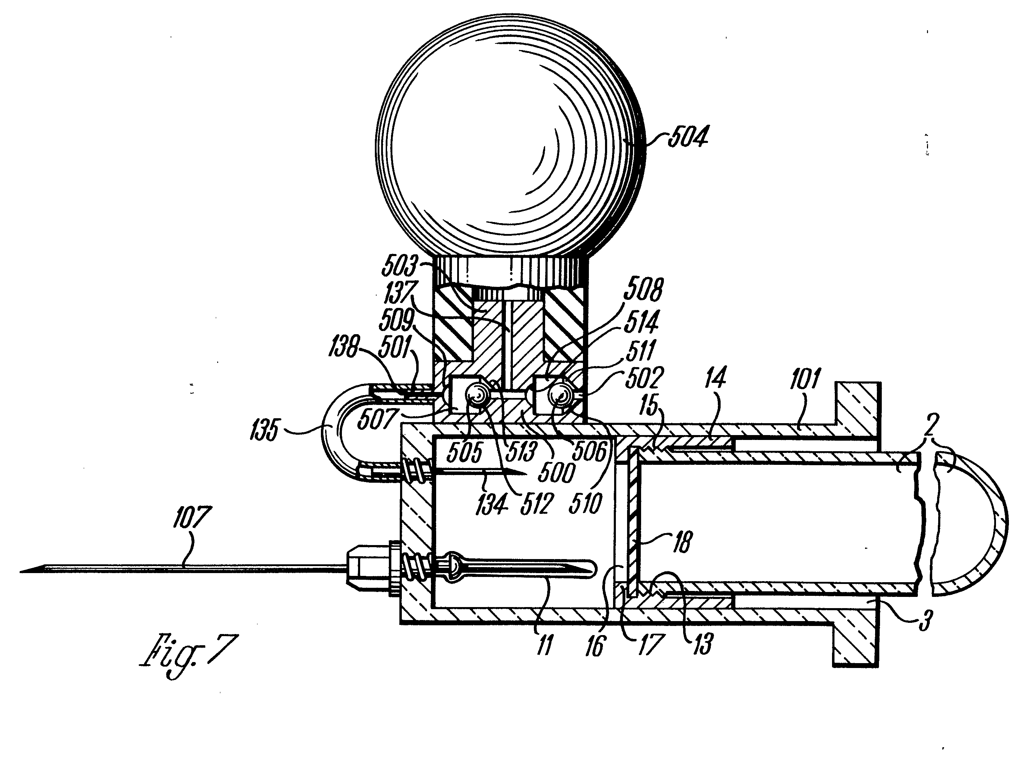

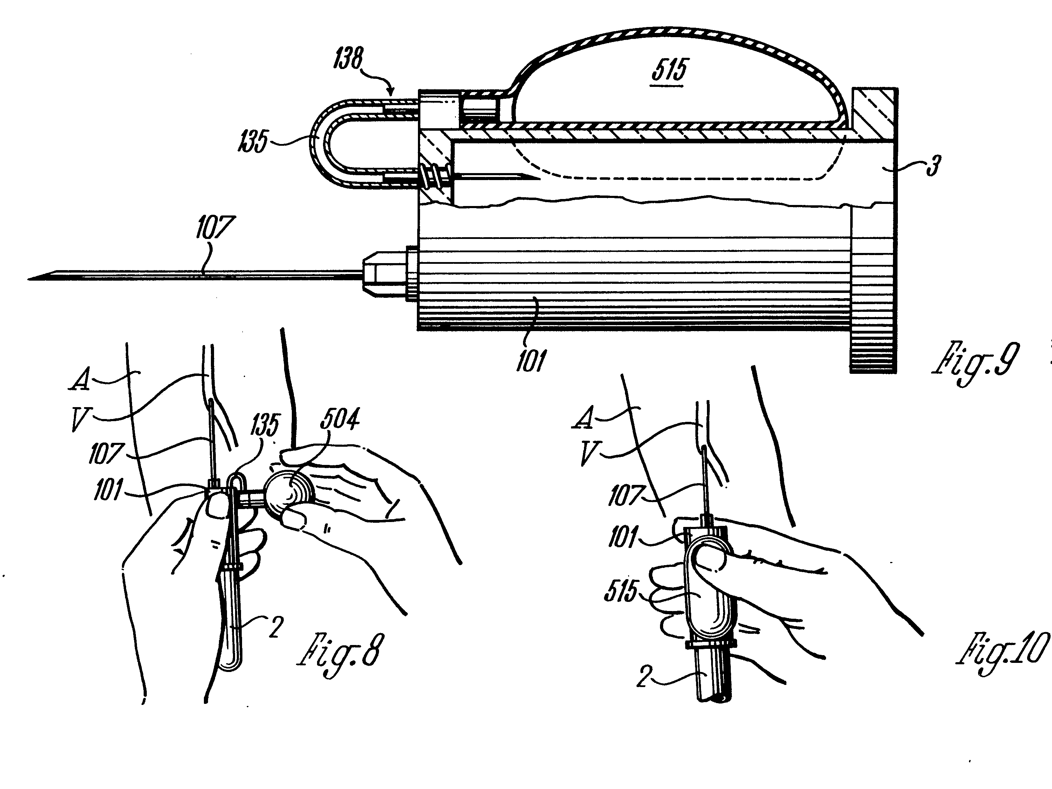

Das Blutentnahmegerät nach Fig. 1 bis 3 wird durch
einen Halter 1 und ein Blutprobenröhrchen 2 gebildet. Der Halter 1 ist von hohlzylindrischer
Form und an seinem einen Ende 3 offen. An seinem
anderen Ende 4 ist der Halter 1 mit einer Gewindebohrung 5 versehen, in die exzentrisch (vgl. Fig.1a)
eine Nadelhalterung 6 eingeschraubt ist. Die Nadelhalterung 6 dient zur festen Aufnahme einer doppelendigen hohlen Nadel 7, deren eines Ende 8 sich in
der gezeitgten Weise außerhalb des Halters 1 befindet und zur Blutentnahme in die Vener einer
Person eingestochen wird, und deren anderes Ende 9 in das Innere des hohlzylindrischen Innenraums 10 des Halters 1 hineinragt. Der sich in den Innenraum 10 hinein erstreckende Teil der Nadel 7 ist von einer Schutzhülle 11 aus leicht durchstechbarem, leichtem, weichem Material umgeben. Am Ende 3 ist der Halter 1 mit einem Flansch 12 versehen, der die Handhabung bei der Blutentnahme erleichtert.

Das Blutprobenröhrchen 2 wird vorzugsweise aus durchsichtigem oder transparentem Kunststoff, z.B. Polystyrol oder Polypropylen, hergestellt und weist einen Schraubverschluß auf, der dadurch gebildet wird, daß auf das (in Fig. 1) linke und mit einem Schraubgewinde 13 versehene Ende des Blutprobenröhrchens 2 eine Kappe 14 aufgeschraubt wird, die mit einem entsprechenden Innengewinde 15 versehen ist. Die Kappe 14 weist ferner eine Öffnung 16 auf, deren Durchmesser so bestimmt ist, daß der sich von außen nach innen und senkrecht zur Achse des Blutprobenröhrchens 2 erstreckende Rand 17 so weit übersteht, daß er eine Membran 18 gegen die offene Stirnseite des Blutprobenröhrchens 2 an dessen offenem (In Fig. 1) linkem Ende drückt. Dadurch wird das Innere des Blutprobenröhrchens 2 abgedichtet, so daß ein Vakuum im Röhrchen aufrechterhalten werden kann. Die Membran 18 ist aus weichem Gummi, der die Eigenschaft hat, daß er sich, wenn man eine Nadel hindurchsticht und sie wieder herauszieht, wieder schließt. Solche Materialien sind bekannt. Die Dicke der Membran beträgt 1,5 bis

0017728

- 8 -

3 mm, vorzugsweise 2 mm.

Die Kappe 14 ist auf ihrer Außenseite zylindrisch ausgebildet derart, daß sie in den hohlzylindrischen Innenraum 10 des Halters 1 hineinpaßt. Das Blutprobenröhrchen 2 kann also mit aufgeschraubter Kappe 14 in den Halter 1 eingeschoben werden (vgl. Fig. 2). Dabei sticht das Ende 9 der Nadel 7 durch die Membran 18 hindurch. Über den inneren Durchgang der hohlen Nadel 7 entsteht so eine Verbinddung des Inneren des Blutprobenröhrchens 2 mit der Öffnung der Nadel 7 an ihrem Ende 8. Danach wird, kurz bevor die Spitze 9 der Nadel 7 in die Membran 18 eindringt, die Schutzhülle 11 an der Spitze 9 der Nadel 7 durchstoßen. Die Schutzhülle 11 wird beim weiteren Einschieben in der Art, wie aus Fig. 2 ersichtlich, balgartig zusammengedrückt.

Die Blutprobenröhrchen 2 werden zunächst in der in Fig. 2 ersichtlichen Weise, in der sie vakuumdicht verschlossen sind, hergestellt. Sie sind dabei jedoch zunächst nicht evakuiert. Das Blutentnahmegerät nach der Erfindung unterscheidet sich also von allen bekannten, mit Vakuum arbeitenden Blutentnahmesystemen dadurch, daß das Blutprobenröhrchen 2 zunächst nicht unter Vakuum stehendem Zustand zur Verfügung gestellt, d.h. in den Handel gebracht und an die Benutzer abgegeben wird.

Erst zeitlich kurz vor der Verwendung, also etwa jeweils zu Beginn eines Tages für einen Tagesbedarf oder unmittelbar vor der Verwendung des Blutentnahmegerätes, wird Vakuum in den bereits vorher dicht geschlossenen Blutprobenröhrchen 2 erzeugt. Dies geschieht in der in Fig. 3 dargestellten Weise: das dicht verschlossene, jedoch noch nicht evakuierte Blutprobenröhrchen 2 wird in einer einfachen Evakuierungseinrichtung 19 evakuiert. Diese Evakuierungseinrichtung 19 wird durch eine hohle Nadel 20 gebildet, die über eine Leitung 21 mit einer Vakuumpumpe 22 verbunden ist. Die Vakuumpumpe 22 wird durch einen Elektromotor 23 angetrieben, dem Strom über die Leitungen 24 zugeführt wird. Die Evakuierungseinrichtung 19 weist ferner, die Nadel umgebend, einen Einführungstrichter 25 auf. Das Blutprobenröhrchen 2 kann in den Einführungstrichter hineingeschoben werden, so daß dabei das Ende 26 der Nadel 20 die Membran 18 durchstößt. Ist das Blutprobenröhrchen 2 so weit eingeschoben, daß der Rand 17 an dem Bund 27, der einen Anschlag bildet, anliegt, so verschiebt er gleichzeitig einen Taster oder Fühler 28 so weit, daß dieser einen mit ihm mechanisch gekoppelten Arbeitskontakt 29 schließt. Dieser Arbeitskontakt liegt in der elektrischen Leitung 24, die bezüglich des Elektromotors 23 so geschaltet ist, daß dieser bei Schließen des Arbeitskontaktes 29 eingeschaltet wird. Beim Einsetzen des Blutprobenröhrchens 2 in den Einführungstrichter 25 wird also die Vakuumpumpe 22 eingeschaltet. Das Blutprobenröhrchen 2 wird evakuiert. Gleichzeitig erfolgt über ein an die Leitung 21 angeschlossenes Druckmeßgerät 30 die Messung des Vakuums in der Leitung 21 und damit auch im Blutprobenröhrchen 2. Ist ein bestimm-

0017728

- 10 -

ter Sollwert erreicht, so schließt sich ein im
Druckmeßgerät vorgesehener Arbeitskontakt 31, der
über ein Relais 32 und dessen Ruhekontakt 33 die
Leitung 24 unterbricht und damit den Elektromotor
23 und mit diesem die Vakuumpumpe 22 abschaltet.
Dann wird das Blutprobenröhrchen 2 wieder von der
Nadel 20 abgezogen. Es ist dann betriebsbereit.

Die Blutentnahme bei einem Patienten geht dann
folgendermaßen vor sich: zunächst nimmt man den
Halter 1, in den das Blutprobenröhrchen nur so weit
eingeschoben ist, daß die Membran 18 noch nicht
durchstochen ist, und sticht die Spitze 8 in die
Vene eines Patienten. Dann schiebt man das Blutprobenröhrchen 2 weiter in den Innenraum 10 ein,
bis die Spitze 9 die Membran 18 durchsticht. Dann
saugt das Vakuum im Blutprobenröhrchen 2 Blut aus
der Vene ein. Ist genügend Blut entnommen, so wird
das Blutprobenröhrchen 2 aus dem Halter 1 entfernt.
Die Membran 18 schließt sich wieder. Der Blutentnahmevorgang ist abgeschlossen. Die Blutprobe kann
dann aus dem Bluprobenröhrchen 2 entnommen werden,
wenn man die Kappe 14 abschraubt.

Ein weiteres Ausführungsbeispiel zeigen die Figuren
4a bis 4c. Der Halter 101 ist mit zwei Nadeln
versehen, nämlich einer ersten Nadel 107 und einer
zweiten Nadel 134, die in eine Hohlleitung 135
übergeht, die auf der Außenseite des Halters 101
zunächst radial nach außen, dann entlang der
zylindrischen Außenwand ein Stück geführt ist. An
das Ende 136 der Hohlleitung 135 ist zur Aspiration bei der Blutentnahme eine Saugleitung 137 mit Hilfe einer Steckverbindung 138 angeschlossen. Das bedeutet: ist (analog zu Fig. 2) ein Blutprobenröhrchen 2 so weit in den zylindrischen Innenraum 110 des Halters 101 eingeschoben, daß die beiden Nadeln 107 und 134 die Membran 18 des Blutprobenröhrchens 2 durchstochen haben, so kann man die Blutentnahme beeinflussen. Saugt man an der Saugleitung, so wird das Vakuum im Blutprobenröhrchen 2 verstärkt; wird Luft eingedrückt, so wird die Blutentnahme beendet. Auf diese Weise ist eine beliebige Steuerung des Blutentnahmevorgangs möglich.

Das beschriebene Blutentnahmesystem wird besonder einfach dadurch, daß das Vakuum in dem Blutentnahmeröhrchen 2 nur kurzzeitig, etwa für die Dauer eines Tages, meist aber weniger, aufrechterhalten werden muß, da das Vakuum in dem Blutentnahmeröhrchen 2 erst relativ kurzzeitig vor der Anwendung des Blutentnahmesystems geschaffen wird (wie anhand von Fig. 3 beschrieben). Das hat zur Folge, daß man sicher sein kann, daß zum Zeitpunkt der Anwendung dieses Blutentnahmesystems ein Vakuum im Blutentnahmeröhrchen 2 gegeben ist und daß dies ferner auch einen ganz bestimmten Wert hat. Damit ist eine bestimmte Geschwindigkeit der Blutentnahme gewährleistet. Die Vakuierung ist mit Hilfe der einfachen, in Fig. 3 beschriebenen Vorrichtung kurz vor der Anwendung äußerst einfach und billig. Die Evakuierungseinrichtung 19 besteht (vom Kostengesichtspunkt her gesehen) im wesentlichen aus der Vakuumpumpe, die relativ billig und einfach ist.

Diese Eigenschaft des erfindungsgemäßen Blutentnahmesystems macht es nun auch möglich, daß von vorn herein die Dichtung, bestimmt durch die Dicke der Membran 18 sowie die Dichtfläche und den Anpreßdruck, nur so ausgelegt wird, daß sie über einen Zeitraum von zwei oder drei Tagen maximal das gewünschte Vakuum aufrechterhält. Das hängt damit zusammen, daß die Erfindung von den seither verwendeten Gummistopfen abgeht und einen einfachen Schraubverschluß verwendet. Er kann geöffnet werden, ohne daß, wie beim Abziehen eines Gummistopfens, ein "Aerosol"-Effekt auftritt.

Beim Ausführungsbeispiel nach Fig. 5 hat der Rand 217 der Kappe 214 auf der Innenseite einen kleinen umlaufenden Steg 239 mit einer relativ scharfen Kante, der sich in die Oberseite der Membran 218 eingräbt und sie dort festhält. Dies kann der Fall sein, wenn die Membran so dünn ist, daß sie im Blutprobenröhrchen 202 nach innen in die strichpunktiert eingezeichnete Position gezogen wird. Die Membran 218 hat in der Mitte an einer Stelle, an der sie von der Nadel 207 durchstochen wird, eine Ausnehmung 240, so daß die Nadel 207 nur noch den dünnen Teil 241 durchstechen muß.

Beim Ausführungsbeispiel nach Fig. 6 ist, im Vergleich zu Fig. 5, die Ausnehmung 340 in der Membran 318 sehr viel größer. Es bleibt nur an deren Rand ein Wulst 341 stehen, in den der Steg 339 an der Kappe 314 eingreift. Wie in Fig. 1a und 4b sitzt die Nadel 307 im Halter 301 exzentrisch. Dies er-

- 13 -

leichtert den Einstich in eine Vene.

Das Blutentnahmegerät nach Fig. 7 stellt eine weitere Ausbildung des Blutentnahmegerätes nach Fig. 4a-4c dar. Es wird gebildet durch einen Halter 101 und ein Blutprobenröhrchen 2.

Gemäß der hier vorliegenden Ausbildung ist die Saugleitung 137 über die Steckverbindung 138 mit einem 3-Wege-Ventil 500 verbunden. Die erste Öffnung ist die Saugleitung 137; die zweite Öffnung 501 mündet in die Steckverbindung 138; die dritte Öffnung 502 geht ins Freie. Auf den Stutzen 503, in dem die Saugleitung 137 vorgesehen ist, ist ein zusammenpreßbarer Gummiballon 504 aufgesteckt. Nach dem Zusammenpressen hat er die Tendenz, wieder die gezeigte runde Form anzunehmen. Man kann damit also eine Pumpwirkung erzeugen, die für die sog. Aspiration, d.h. die Blutentnahme bei gleichzeitiger Erzeugung zusätzlichen Unterdrucks im Blutprobenröhrchen 2, eingesetzt werden kann.

Das Ventil 500 ist im Ausführungsbeispiel als Doppel-Kugelventil ausgebildet mit zwei Kugeln 505 und 506, die in Ventilkammern 507 und 508 angeordnet sind. Wird der Gummiballon 504 zusammengedrückt, so entsteht in der Saugleitung 137 zunächst Überdruck. Die Kugel 505 in der linken Kammer 507 wird auf den Ventilsitz 509 gedrückt und verschließt diesen. Es kann also keine Luft in die Leitung 135 und damit in das Blutprobenröhrchen 2 austreten. Gleichzeitig wird auch die rechte Kugel 506 in der

- 14 -

Kammer 508 auf den rechten Ventilsitz 510 gedrückt.
Doch ist dieser Ventilsitz nicht dicht ausgebildet,
sondern weist Schlitze 511 auf, durch die die
Luft dann entweichen und über die Öffnung 502 ins
Freie austreten kann. Beim Zusammendrücken des
Gummiballons 504 ist also die Öffnung 501 und damit
zur Nadel 134 versperrt; die beim Zusammenpressen
ausgestoßene Luft entweicht über die Öffnung 502
ins Freie.

Läßt man nun den zusammengedrückten Gummiballon 504
los, so entsteht in der Saugleitung 137 Unterdruck.
Die beiden Kugeln 505 und 506 werden nach innen
gezogen. Die Kugel 505 sitzt dabei am Ventilsitz
512 auf. Durch die beiden Schlitze 513 kann dennoch
Luft von der Leitung 501 her in die Saugleitung 137
übertreten. Der Unterdruck bzw. die Saugwirkung
werden also im Blutprobenröhrchen 2 voll wirksam.
Dagegen verschließt die Kugel 506 den Ventilsitz
514, so daß die Öffnung 502 versperrt ist.

Auf diese Weise kann man durch von Hand kontrolliertes Nachlassen des Gummiballons 504 den Unterdruck kontrollieren, der im Blutprobenröhrchen 2
entsteht.

Es ist ausdrücklich darauf hinzuweisen, daß das
gezeigte Ventil 500 lediglich ein Ausführungsbeispiel ohne einschränkende Bedeutung darstellt. Es
sind hier alle Ventile einsetzbar, die gewährleisten, daß über die Hohlleitung 135 und die
zweite Nadel 134 in dem Blutprobenröhrchen 2 ledig-

- 15 -

lich Unterdruck wirksam wird und beim Zusammenpressen des Gummiballons keine Luft in das Blutprobenröhrchen 2 gedrückt wird.

Wie in Fig. 8 dargestellt, kann der Gummiballon 504
bei der Verwendung des Blutentnahmegerätes zur
Blutentnahme aus einer Vene V eines Armes A auch
als Halter zur leichteren Handhabung genutzt werden.

Das Ausführungsbeispiel nach Fig. 9 unterscheider
sich von dem nach Fig. 7 dadurch, daß der Gummiballon 515 als Fischblase ausgebildet ist und an
dem Halter 101 anliegt, so daß sich die Handhabung
mit nur einer Hand in besonders einfacher Weise
ergibt. Dies ist in Fig. 10 dargestellt.


- Ende der Beschreibung -

0017728

- 16 -

Patentansprüche

1. Blutentnahmegerät mit einem hohlzylindrischen Halter, in dessen einem Ende eine doppelendige hohle Nadel angeordnet und in dessen anderes, offenes Ende ein mit einer Dichtung verschlossenes Blutprobenröhrchen so weit einschiebbar ist, daß das in den hohlzylindrischen Innenraum des Halters hineinragende eine Ende der Nadel die Dichtung durchstößt und eine Verbindung des Innenraums des Blutprobenröhrchens mit dem außerhalb des Halters befindlichen anderen Ende der Nadel herstellt, dadurch gekennzeichnet, daß das Blutprobenröhrchen (2) durch eine aufschraubbare Kappe (14) verschlossen wird, die eine Öffnung (16) aufweist, deren Rand (17) eine die Dichtung bildende Membrane (18) aus einem Material, das vom einen Ende (9) der Nadel (7) durchstechbar ist, an das offene Ende des Blutprobenröhrchens (2) abdichtend andrückt.

2. Blutentnahmegerät nach Anspruch 1, dadurch gekennzeichnet, daß die Dicke der Membran (18) 1,5 bis 3 mm, vorzugsweise 2 mm beträgt.

- 17 -

3. Blutentnahmegerät nach Anspruch 1 oder 2, dadurch gekennzeichnet, daß gegenüber der Nadel (207) in der Membran eine Ausnehmung (240) vorgesehen ist.

4. Blutentnahmegerät nach Anspruch 1 oder einem der folgenden, dadurch gekennzeichnet, daß in der Kappe (213, 314) Mittel (239, 339) zum Festhalten der Membran (218, 318) vorgesehen sind.

5. Blutentnahmegerät nach Anspruch 1 oder einem der folgenden, dadurch gekennzeichnet, daß die Nadel (307) im Halter (301) exzentrisch und gegenüber einer Ausnehmung (340) in der Membran (318) angeordnet ist und daß die Ausnehmung (340) in der Membran so groß ist, daß lediglich ein Wulst (341) zur Abdichtung zwischen Blutprobenröhrchen (302) und Kappe (314) am Rande der Ausnehmung stehen bleibt.

6. Blutentnahmegerät nach Anspruch 1 oder einem der folgenden, dadurch gekennzeichnet, daß die Nadel (7) am Halter (1) exzentrisch angeordnet ist.

7. Blutentnahmegerät nach einem der Ansprüche 1 bis 6, dadurch gekennzeichnet, daß der Halter (101) eine zweite, bei Einschieben des Blutprobenröhrchens (2) in den hohlzylindrischen Innenraum (110) die Membran (18) durchstoßende Nadel (134) aufweist, die über eine an der Außenseite des Halters (101) geführte Hohlleitung (135) mit einer Druck- und/oder Saugleitung (137) zur Aspiration verbindbar ist.

8   Blutentnahmegerät nach Anspruch 1 oder einem der folgenden, dadurch gekennzeichnet, daß das Blutentnahmeröhrchen (2) bei aufgeschraubter Kappe und abdichtend eingesetzter Membran (18) nicht evakuiert ist.

9. Verfahren zur Evakuierung eines Röhrchens nach Anspruch 8, dadurch gekennzeichnet, daß die Evakuierung vor der Blutentnahme mit Hilfe einer Nadel (20, 26) erfolgt, die mit einer Vakuumquelle (22) in Verbindung steht und die durch die Membran (18) hindurchgestoßen wird.

10. Verfahren nach Anspruch 9, dadurch gekennzeichnet, daß die Evakuierung bei Erreichen eines bestimmten Sollwertes durch Schalteinrichtungen (32, 33) beendet wird.

11. Blutentnahmegerät nach Anspruch 7, dadurch gekennzeichnet, daß die Saugleitung (137) mit einem Gummiballon (504) verbunden ist.

12. Blutentnahmevorrichtung nach Anspruch 11, dadurch gekennzeichnet, daß der Gummiballon (504) als Handgriff ausgebildet und im wesentlichen senkrecht zur Hauptrichtung des Halters (101) angeordnet ist.

0017728

- 19 -

13. Blutentnahmevorrichtung nach Anspruch 1,
dadurch gekennzeichnet, daß der Gummiballon
(504) mit der Form einer Fischblase entlang
der Außenseite des Halters (101) an diesem
zumindest teilweise anliegend ausgebildet ist.

14. Blutentnahmegerät nach Anspruch 1 oder einem
der folgenden, dadurch gekennzeichnet, daß
zwischen dem Gummiballon (504) und der die
Verbindung mit der zweiten Nadel (134) herstellenden Leitung (135) ein Ventil (500)
vorgesehen ist, das die Verbindung zum Blutprobenröhrchen (2) beim Zusammendrücken des
Gummiballons (504) sperrt und bei Loslassen
des Gummiballons freigibt.

- Ende der Ansprüche -

Fig.1

Fig.2

Fig.1a

Fig.3

1/4

0017728

0017728

Fig.4a

Fig.4b

Fig.4c

Fig.5

Fig.6

Fig.7

Fig.9

Fig.8

Fig.10

4/4

0017728